# EUROPÄISCHE PATENTSCHRIFT

(11) **EP 2 954 798 B1**
(45) Veröffentlichungstag und Bekanntmachung des Hinweises auf die Patenterteilung: **12.04.2017**
(21) Anmeldenummer: 14172025.0
(22) Anmeldetag: 11.06.2014
(51) Int. Cl.: A43D 1/02

(54) **Messverfahren zur Bestimmung biometrischer Daten menschlicher Füße**
Measuring method for determining biometric data of human feet
Procédé de mesure pour determiner des données biométriques de pieds humains

(43) Veröffentlichungstag der Anmeldung: 16.12.2015
(73) Patentinhaber: VITRONIC Dr.-Ing. Stein Bildverarbeitungssysteme GmbH, 65189 Wiesbaden (DE)
(72) Erfinder: Alfaro Arrieta, Alejandro, 68199 Mannheim (DE); Hoffmann, Burghard, 65232 Taunusstein (DE); Bürgel, Udo, 65201 Wiesbaden (DE); Jurca, Ales, 5281 Spodnja Idrija (SI)
(74) Vertreter: Neumann Müller Oberwalleney & Partner Patentanwälte

(56) Entgegenhaltungen:
- EP-A1- 2 641 539
- WO-A1-2013/071416
- DE-A1-102011 121 086
- US-B1- 6 546 356
- US-B1- 6 549 639

## Beschreibung

Die vorliegende Erfindung betrifft ein Messverfahren zur Bestimmung biometrischer Daten menschlicher Füße.

Unter biometrischen Daten werden allgemein solche Daten verstanden, die sich im Rahmen der Körpermessung von Lebewesen bestimmen lassen. Am Beispiel des menschlichen Fußes können die biometrischen Daten sowohl die Fußlänge, die Fußbreite und die Fußhöhe als auch eine dreidimensionale Darstellung des Fußes umfassen.

Aus der DE 10 2011 121 086 A1 ist ein Messverfahren zur Bestimmung der Fußlänge und der Breite des Vorderfußes bekannt. Ein zu vermessender Fuß wird auf ein weißes DIN A4-Blatt gestellt, wobei das in DIN A4-Blatt ein mit einem Tintenstrahl - oder Laserdrucker- aufgedrucktes Referenzmuster aufweisen kann. Mit einem Smartphone oder einem Tablet-Computer kann ein Bild des platzierten Fußes aufgenommen werden.

Die WO 2013/071416 A1 offenbart ein weiteres Messverfahren, bei dem ein dreidimensionales Modell eines menschlichen Fußes mittels mit einem Triangulationsscanner aufgenommener Bilder erstellt werden kann.

Ein Messsystem zur Bestimmung der Länge und der Breite eines menschlichen Fußes ist beispielsweise aus der DE 10 2011 116 727 A1 bekannt. Dieses Messsystem weist eine beleuchtete Bodenplatte mit einem geometrischen bodenseitigen Referenzmuster auf. Ferner ist eine frei bewegliche Kamera vorgesehen, um einen auf der Bodenplatte platzierten Fuß zu fotografieren.

Als nachteilig wird empfunden, dass das bekannte Messsystem lediglich geeignet ist, die Länge oder die Breite eines menschlichen Fußes zu bestimmen.

Aus der DE 10 2011 007 678 A1 ist ein Messsystem bekannt, auf dessen Bodenplatte Markierungslinien zur Längsausrichtung des zu vermessenden Fußes aufgebracht sind. An einem Ende der Bodenplatte ragt ein Anschlag ab, gegen den die Ferse des Fußes angesetzt wird. Am gegenüberliegenden Ende der Bodenplatte ist eine Bildaufnahmevorrichtung starr gehalten, die von schräg oben Bilder des zu vermessenden Fotos aufnimmt. Um mit einer Bildaufnahme zwei Ansichten des Fußes zu erhalten, ist im Blickfeld der Bildaufnahmevorrichtung ein Spiegelsystem vorgesehen.

Ein weiteres ähnliches Messsystem ist aus der AU 2004 0 202 110 A1 bekannt. Das bekannte Messsystem weist eine mit Zentimeter-Markierungen versehene Bodenplatte auf, auf welcher ein zu vermessender Fuß ausgerichtet wird. An der Bodenplatte ragen seitlich Spiegel ab. An der Bodenplatte ist eine Bildaufnahmevorrichtung befestigt, die starr auf die Bodenplatte und die Spiegel ausgerichtet ist.

Aus der US 6,546,356 B1 ist ein Podest zur Vermessung eines menschlichen Fußes bekannt, das sowohl auf einer Bodenplatte als auch auf einem Wandelement jeweils ein geometrisches Referenzmuster aufweist.

An diesen Ausgestaltungen wird als nachteilig empfunden, dass der zu vermessende Fuß zunächst ausgerichtet werden muss. Zudem ist das Messsystem durch die starr an der Bodenplatte befestigte Bildaufnahmevorrichtung und das Spiegelsystem groß bauend und unhandlich in der Bedienung.

Aufgabe der vorliegenden Erfindung ist es daher, ein Messverfahren zur Bestimmung biometrischer Daten menschlicher Füße bereitzustellen, mit dem über die Länge und die Breite hinaus weitere biometrische Daten der Füße auf einfache Weise bestimmt werden können.

Diese Aufgabe ist erfindungsgemäß bei einem Messverfahren der eingangs genannten Art dadurch gelöst, dass das Messverfahren die folgenden Schritte umfasst: Bereitstellen eines Referenzobjektes, das wenigstens ein Referenzmuster aufweist; Bereitstellen einer frei beweglichen Bildaufnahmevorrichtung; Automatisches Erkennen des relativen Standortes und der Ausrichtung der Bildaufnahmevorrichtung zum Referenzobjekt anhand des wenigstens einen Referenzmusters; Automatisches Aufnehmen mehrerer Bilder durch die Bildaufnahmevorrichtung, wobei jedes einzelne Bild nur dann aufgenommen wird, wenn der relative Standort und die Ausrichtung der Bildaufnahmevorrichtung zum Referenzobjekt einer für das jeweilige Bild vorgegebenen Auslösestellung entspricht; Bestimmung biometrischer Daten des wenigstens einen Fußes mittels der aufgenommenen Bilder.

Wenn die Bildaufnahmevorrichtung zumindest grob auf das Referenzobjekt ausgerichtet ist, erkennt diese anhand des wenigstens einen Referenzmusters ihren relativen Standort und ihre Ausrichtung zum Referenzobjekt. Mit anderen Worten erkennt die Bildaufnahmevorrichtung anhand des wenigstens einen Referenzmusters insbesondere ihren Abstand, ihre Höhe und ihren Blickwinkel zum Referenzobjekt. Somit kann die Bildaufnahmevorrichtung selbstständig ihren Standort und ihre Ausrichtung in Relation zum Referenzobjekt bestimmen und die Bilder automatisch aufnehmen, wenn insbesondere ein Benutzer diese in den vorgegebenen Auslösepunkten hält. Üblicherweise werden zur Generierung eines dreidimensionalen Modells mindestens zwei Bilder pro Fuß unterschiedlicher Ansichten benötigt, so dass für jeden zu vermessenden Fuß wenigstens zwei Auslösepunkte vorgegeben sein können. Beispielsweise kann ein erster Auslösepunkt durch eine Position vor dem Referenzobjekt, das heißt in Verlängerung des wenigstens einen Fußes, und eine weitere Position seitlich vom Referenzobjekt mit jeweils vorgegebenen Abständen zum Referenzobjekt definiert sein.

In bevorzugter Weise umfasst der Schritt der Bestimmung der biometrischen Daten des wenigstens einen Fußes mittels der aufgenommenen Bilder zumindest die folgenden Teilschritte: Generierung zumindest eines dreidimensionalen Modells wenigstens eines auf dem Referenzobjekt platzierten Fußes auf Basis der aufgenommenen Bilder durch die Verarbeitungsvorrichtung; und Bestimmung der biometrischen Daten des wenigstens einen Fußes anhand des zumindest einen dreidimensionalen Modells durch die Verarbeitungsvorrichtung.

Zweckmäßigerweise wird ein Podest als das Referenzobjekt im Rahmen des Verfahrens verwendet. Das Podest eignet sich besonders gut für computerunterstütze Bildanalyseverfahren zur Berechnung von dreidimensionalen Modellen des wenigstens einen Fußes.

Weiterhin kann das beschriebene Messsystem zur Durchführung des Messverfahrens verwendet werden. Hierzu wird der wenigstens eine zu vermessende menschliche Fuß auf das bodenseitige Referenzmuster des Podestes platziert. Die frei bewegliche Bildaufnahmevorrichtung erkennt automatisch ihren relativen Standort und ihre Ausrichtung zum Podest anhand des wenigstens einen bodenseitigen Referenzmusters und/oder des wenigstens einen wandseitigen Referenzmusters. Dabei nimmt die Bildaufnahmevorrichtung automatisch ein Bild auf, wenn ihr relativer Standort und ihre Ausrichtung zum Podest einer für das jeweilige Bild vorgegebenen Auslösestellung entspricht.

Gemäß einem Aspekt des erfindungsgemäßen Messverfahrens ist weiterhin folgender Schritt vorgesehen: Ausgabe von Anweisungen zur Führung der Bildaufnahmevorrichtung zur jeweiligen Auslösestellung. Damit der Benutzer der frei beweglichen Bildaufnahmevorrichtung weiß, wo sich die vorgegebenen Auslösepunkte befinden, gibt diese Anweisungen aus, um den Benutzer zu einer aktiven Führung der Bildaufnahmevorrichtung in eine der Auslösestellungen zu bewegen. Die Bildaufnahmevorrichtung kann zusätzlich oder alternativ eine Übersicht sämtlicher Auslösestellungen in Relation zum Referenzobjekt und insbesondere ihren aktuellen Standort anzeigen, damit der Benutzer schneller die einzelnen Auslösestellungen finden kann.

Zweckmäßigerweise werden vor der Ausgabe der jeweiligen Anweisung zur Führung der Bildaufnahmevorrichtung zur jeweiligen Auslösestellung folgende Schritte durchgeführt: Automatisches Erkennen des relativen Standorts und der Ausrichtung der Bildaufnahmevorrichtung zum Referenzobjekt anhand des wenigstens einen Referenzmusters; Berechnen eines Weges vom erkannten relativen Standort mit der erkannten Ausrichtung zur jeweiligen Auslösestellung. Die Anweisungen können Befehle wie die Bildaufnahmevorrichtung näher an das beziehungsweise weiter weg vom Referenzobjekt bewegen, nach links beziehungsweise rechts schwenken, nach oben beziehungsweise unten schwenken, im Uhrzeigersinn oder gegen den Uhrzeigersinn um das Referenzobjekt bewegen, höher beziehungsweise tiefer zu halten, umfassen.

Vorteilhafterweise erfolgen die Anweisungen zur Führung des Benutzers über optische oder akustische Anzeigemittel. Beispielsweise kann die Bildaufnahmevorrichtung oder die Ausgabevorrichtung ein Display und/oder einen Lautsprecher aufweisen, über welchen die Anweisungen ausgegeben werden. In bevorzugter Weise werden bei einer optischen Darstellung einfache bildliche Symbole, wie beispielsweise Pfeile, verwendet, um den Benutzer zur aktiven Führung der Kamera in die jeweiligen Auslösepunkte aufzufordern.

Gemäß einem weiteren Aspekt des erfindungsgemäßen Messverfahrens ist weiterhin wenigstens einer der folgende Schritte vorgesehen: Abgleichung der bestimmten biometrischen Daten des wenigstens einen Fußes mit Datensätzen von Schuhen, die zumindest Angaben über Schuhgrößen oder Schuhformen diverser Schuhe umfassen, durch die Auswertevorrichtung; Übermittlung einer Trefferliste mit zu den biometrischen Daten passenden Schuhen von Auswertevorrichtung an die Ausgabevorrichtung; und Wiedergabe von Informationen zu den in der Trefferliste aufgeführten Schuhen.

Durch das quer zum bodenseitigen Referenzmuster ausgerichtete wandseitige Referenzmuster des Podestes wird ein ein räumliches Referenzmustersystem aufgespannt, in dem wenigstens ein zu vermessender menschlicher Fuß platziert werden kann. Somit können nicht nur zweidimensionale, sondern auch dreidimensionale biometrische Daten des wenigstens einen Fußes auf einfache Weise bestimmt werden. Durch das dreidimensionale Referenzmustersystem wird ein rückführbares Bezugssystem bereitgestellt, so dass der wenigstens eine zu vermessende Fuß auf einfache Weise auf dem Podest platziert werden kann, ohne dass insbesondere die Ferse des Fußes an einem Anschlag angesetzt werden muss. Des Weiteren ist das Podest besonders gut für computerunterstütze Bildanalyseverfahren zur Berechnung von dreidimensionalen Modellen des wenigstens einen Fußes geeignet. Die Referenzmuster können beispielsweise als Punkt-Marker beziehungsweise Kalibriermarkungen oder als regelmäßige Linienraster gestaltet sein. Neben menschlichen Füßen können ebenso die biometrischen irgendeines anderen Körperteils eines Lebewesens, beispielsweise einer menschlichen Hand oder eines tierischen Körperteils, bestimmt werden. Dabei können alle biometrischen Daten von Körperteilen von Lebewesen erfasst werden, die sich zwei- oder dreidimensional wiedergegeben und auf dem erfindungsgemäßen Podest platzieren lassen.

In bevorzugter Weise sind das Wandelement und die Bodenplatte in einem Winkel zueinander angeordnet, der im Bereich zwischen 70 und 140 Grad liegt. Dabei handelt es sich um den Winkel, der die zum Fuß weisende Oberseite der Bodenplatte und die zum Fuß weisende Außenseite des Wandelements einschließt. Um die Höhe des Fußes einfach und genau bestimmen zu können, ist das Wandelement vorzugsweise rechtwinklig zur Bodenplatte angeordnet. In vorteilhafter Weise ist das Wandelement unmittelbar an der Bodenplatte befestigt.

Die Bodenplatte und/oder das Wandelement können eine retroreflektierende Oberfläche aufweisen. Das heißt die einfallende Strahlung wird weitgehend unabhängig von der Ausrichtung der retroreflektierenden Oberfläche zumindest Großteils in Richtung zurück zur Strahlungsquelle reflektiert. Diese eignet sich besonders für solche Fälle, in denen der wenigstens eine zu vermessende Fuß fotografiert wird, um die biometrischen Daten mittels Bildanalyseverfahren an einem Computer zu bestimmen. Durch die retroreflektierende Oberfläche wird der wenigstens eine zu vermessende Fuß bei Verwendung eines Blitzlichtes kontrastreich als dunkle Fläche auf den Bildern dargestellt. Somit wird der Bildkontrast der Bildaufnahme erhöht, so dass sich im Bildanalyseverfahren der wenigstens eine zu vermessende Fuß besser vom Podest trennen lässt. In einfachster Weise wird die retroreflektierende Oberfläche durch eine Folie bereitgestellt, die auf die Oberfläche der Bodenplatte aufgeklebt ist. Das geometrische bodenseitige und/oder das wandseitige Referenzmuster kann/können beispielsweise auf der Folie oder im Falle einer transparenten Folie unmittelbar auf der Bodenplatte aufgebracht sein. Weiterhin kann es sich bei der retroreflektierenden Oberfläche der Bodenplatte und/oder des Wandelements zum Beispiel um eine obere Schicht der Bodenplatte beziehungsweise des Wandelements handeln.

Weiterhin kann wenigstens ein quer zur Bodenplatte angeordneter Spiegel vorgesehen sein, wobei eine reflektierende Spiegelfläche des Spiegels dem wandseitigen

Referenzmuster gegenüberliegend angeordnet ist. In bevorzugter Weise sind der wenigstens eine Spiegel und die Bodenplatte in einem Winkel zueinander angeordnet, der im Bereich zwischen 70 und 160 Grad liegt. Somit können bei der Bildaufnahme mit einer Kamera mit einem Bild gleichzeitig mehrere Ansichten des wenigstens einen Fußes aufgenommenen werden. Beispielsweise ist neben der Draufsicht auf den wenigstens einen Fuß gleichzeitig die Spiegelseitenansicht des wenigstens einen Fußes erfassbar.

Um mit dem Podest auf einfache Weise zwei Füße zeitgleich platzieren und deren biometrische Daten bestimmen zu können, kann das Podest zu einer durch das Wandelement aufgespannten Ebene symmetrisch ausgebildet sein. Dadurch unterteilt das Wandelement das Podest in zwei symmetrische Hälften, so dass die Bodenplatte auf beiden mit den zu vermessenden Füßen in Kontakt kommenden Oberflächen ein bodenseitiges Referenzmuster und das Wandelement an beiden zu den Füßen weisenden Außenseiten ein wandseitiges Referenzmuster aufweisen.

Weiterhin ist ein Messsystem zur Bestimmung biometrischer Daten menschlicher Füße vorgesehen, das neben dem vorbeschriebenen Podest eine frei bewegliche Bildaufnahmevorrichtung zur Aufnahme mehrerer Bilder des wenigstens einen platzierten Fußes umfasst. Die als tragbares Handgerät ausgebildete Bildaufnahmevorrichtung kann beispielsweise ein Tablet-Computer oder ein Smartphone sein.

Zweckmäßigerweise ist vorgesehen, dass das Messsystem eine Verarbeitungsvorrichtung, die derart ausgebildet ist, dass aus den Bildern ein dreidimensionales Modell des wenigstens einen Fußes generierbar und anhand des Modells biometrische Daten des wenigstens einen Fußes bestimmbar sind, umfasst. Dabei kann das Modell beispielsweise ein auf einer Punktewolke basierendes rudimentäres dreidimensionales Modell des zu vermessenden Fußes sein. Ebenso kann das Modell eine zweidimensionale Draufsicht auf den Fuß und eine zweidimensionale Seitenansicht des Fußes umfassen, die zu einem vereinfachten dreidimensionalen Modell zusammengefasst werden. Es ist auch denkbar, dass das Modell eine dreidimensionale Wiedergabe der Oberfläche des zu vermessenden Fußes umfasst.

Weiterhin kann das Messsystem eine Auswertevorrichtung, die derart ausgebildet ist, dass die ermittelten biometrischen Daten mit Datensätzen von Schuhen anhand hinterlegter Schuhgrößen und/oder Schuhformen abgleichbar und die zu den ermittelten biometrischen Daten passenden Schuhe identifizierbar sind, aufweisen.

Des Weiteren kann das Messsystem eine Ausgabevorrichtung, die derart ausgebildet ist, dass Informationen zu den passenden Schuhen wiedergebbar sind, umfassen.

In bevorzugter Weise ist vorgesehen, dass die Bildaufnahmevorrichtung und die Ausgabevorrichtung eine tragbar ausgebildete bauliche Einheit bilden und die Verarbeitungsvorrichtung sowie die Auswertevorrichtung baulich von der Bildaufnahmevorrichtung und der Ausgabevorrichtung getrennt sind. Vorzugsweise werden die Verarbeitungsvorrichtung und die Auswertevorrichtung durch eine Recheneinheit gebildet, die in einem zentralen Server, der über eine Internetleitung mit der Bildaufnahmevorrichtung kommuniziert, vorgesehen sind. Die von der Bildaufnahmevorrichtung erstellten Bilder können über eine Internetverbindung an die Verarbeitungsvorrichtung übermittelt werden, die anhand der Bilder ein Modell des wenigstens einen Fußes berechnet. Wenn Weiterhin könnte die Auswertevorrichtung anhand des wenigstens einen erstellten dreidimensionalen Modells des wenigstens einen Fußes passende Schuhe anhand hinterlegter dreidimensionaler Schuhmodelle oder insbesondere anhand der berechneten Fußlänge, Fußbreite und/oder Fußhöhe identifizieren. Die identifizierten Schuhe können dann in einer Trefferliste zusammengefasst an die Ausgabevorrichtung übermittelt werden, um die passenden Schuhe dem Kunden zu präsentieren. Neben der vorbeschriebenen baulichen Zusammenfassung der Bildaufnahmevorrichtung und der Ausgabevorrichtung zu einer Einheit sind auch andere Kombinationen möglich. Ebenso können die Bildaufnahmevorrichtung, die Verarbeitungsvorrichtung, die Auswertevorrichtung und die Ausgabevorrichtung als Einzelgeräte baulich voneinander getrennt oder zusammen in einem multifunktionalen Gerät, insbesondere einem tragbaren Handgerät, integriert sein.

Ein bevorzugtes Ausführungsbeispiel der Erfindung wird in den Zeichnungen dargestellt und nachstehend beschrieben. Hierin zeigt:

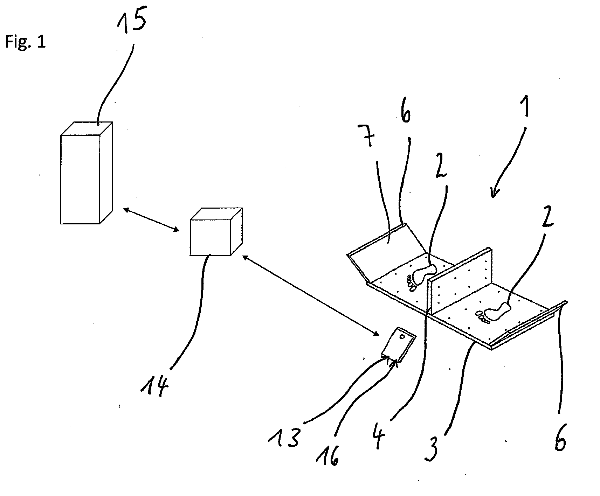

- Figur 1: ein Messsystem in schematischer perspektivischer Darstellung und
- Figur 2: ein Teilausschnitt des Messsystems aus Figur 1 in schematischer perspektivischer Darstellung.

In der Zeichnung ist ein Messsystem dargestellt. Das Messsystem dient der Erstellung dreidimensionaler Modelle zur Bestimmung biometrischer Daten menschlicher Füße. Mit Hilfe des Messsystems können beispielsweise Schuhverkäufer oder Online-Plattformen einem Kunden die für seine individuelle Fußform passenden Schuhe vorschlagen.

Zu dem Messsystem gehört ein Podest 1, auf das eine Person mit beiden Füßen 2, die hier über zwei Fußabdrücke vereinfacht dargestellt sind, treten kann. Konkret weist das Podest 1 eine rechteckige Bodenplatte 3 auf, auf welche die beiden Füße 2 zeitgleich platziert werden können. Damit die Person einen sicheren Stand einnehmen kann, ist die Bodenplatte 3 horizontal ausgerichtet. Weiterhin ist ein rechteckiges Wandelement 4 vorgesehen, dass rechtwinklig zur Bodenplatte 3 angeordnet und an dieser befestigt ist. Das Wandelement 4 ist quer zur Längserstreckung der Bodenplatte 3 ausgerichtet und spannt eine Ebene E auf, zu der das gesamte Podest 1 symmetrisch ausgebildet ist. Mit anderen Worten unterteilt das Wandelement 4 die Bodenplatte 3 in zwei symmetrische zueinander ausgebildete Hälften, auf die links beziehungsweise rechts vom Wandelement 4 jeweils einer der beiden Füße 2 platziert werden kann. An beiden außenliegenden Längsenden 5 der Bodenplatte 3 ist jeweils ein Spiegel 6 an der Bodenplatte 3 gehalten, der parallel zum Wandelement 4 angeordnet ist und diesem mit seiner reflektierenden Spiegelfläche 7 gegenüberliegt. Die Spiegel 6 sind mit etwa 30 Grad aus der Vertikalen nach außen gekippt, so dass der jeweilige Spiegel 6 und die Bodenplatte 3 in einem Winkel von etwa 120° zueinander angeordnet sind.

Auf mit den zu vermessenden Füßen 2 in Kontakt kommenden Oberflächen 8 der Bodenplatte 3 und auf zu den Füßen 2 weisenden Außenseiten 11 des Wandelements 4 sind jeweils eine retroreflektierende Beschichtung 9 vorgesehen.

Um die biometrischen Daten der Füße 2 bestimmen zu können, weist die retroreflektierende Beschichtung 9 auf beiden Hälften der Bodenplatte 3 jeweils ein geometrisches bodenseitiges Referenzmuster 10 auf. Weiterhin weist das Wandelement 4 an den beiden zu den Spiegeln 7 weisenden Außenseiten 11 jeweils ein geometrisches wandseitiges Referenzmuster 12 auf. Jedes der Referenzmuster 10, 12 ist hier aus einer Vielzahl von Referenzpunkten gebildet, die auf einer Umfangslinie eines gedachten Rechteckes angeordnet sind.

Des Weiteren weist das Messsystem eine frei bewegliche, handgehaltene Bildaufnahmevorrichtung 13 auf, mittels derer mehrere digitale Fotos der auf dem Podest 1 platzierten Füße 2 aufgenommenen werden können. Bei der Bildaufnahmevorrichtung 13 kann es sich beispielsweise um einen handelsüblichen Tablet-PC oder ein Smartphone handeln, welches vorzugsweise über ein Blitzgerät verfügt. Durch die retroreflektierenden Oberfläche 9 der Bodenplatte 3 und des Wandelements 4 sind bei Bildaufnahmen mit Blitzlicht die Konturen der Füße 2 deutlich erkennbar.

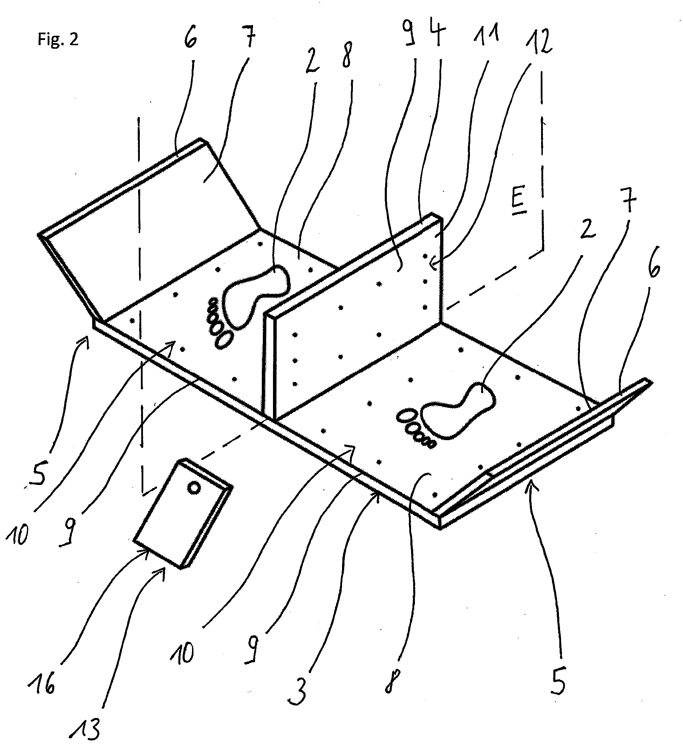

Zur Berechnung der dreidimensionalen Modelle der beiden Füße 2 sind mehrere Ansichten von jedem der auf dem Podest 1 platzierten Füße 2 notwendig. Durch die Spiegel 6 kann die Anzahl der notwendigen Anzahl allerdings reduziert werden, da mit einer Bildaufnahme gleichzeitig mehrere Ansichten des wenigstens einen Fußes 2 aufgenommenen werden können. Beispielsweise ist neben der in Figur 2 gezeigten Draufsicht von schräg vorne auf den linken Fuß 2 gleichzeitig die Spiegelseitenansicht des linken Fußes 2 erfassbar.

Mit der Bildaufnahmevorrichtung 13 werden zunächst Bilder des ersten Fußes 2 und anschließend des zweiten Fußes 2 aus jeweils mehreren vorgegebenen Auslösestellungen, insbesondere aus mindestens zwei Auslösestellungen je Fuß 2, aufgenommen. Die unterschiedlichen Auslösestellungen sind anhand des relativen Standortes und der Ausrichtung der Bildaufnahmevorrichtung 13 zum Podest 1 zum einen für den linken und zum anderen für den rechten Fuß 2 definiert, wodurch eine Rückprojizierung in ein dreidimensionales Weltkoordinatensystem ermöglicht wird. In Figur 2 ist exemplarisch eine der beiden Auslösestellungen für den linken Fuß 2 dargestellt.

Damit ein Benutzer, beispielsweise der Schuhverkäufer, weiß, wo sich die vorgegebenen Auslösestellungen für die Aufnahmen des linken beziehungsweise rechten Fußes 2 befinden, weist die Bildaufnahmevorrichtung 13 eine Software zur automatischen Erkennung ihres relativen Standortes und ihrer Ausrichtung zum Podest 1 anhand der Referenzmuster 10, 12 auf. Befindet sich die Bildaufnahmevorrichtung 13 in einer der vorgegebenen Auslösestellungen für eines der Bilder, löst diese automatisch aus. Befindet sich die Bildaufnahmevorrichtung 13 dagegen nicht in einer der vorgegebenen Auslösestellungen, gibt diese Anweisungen an den Benutzer aus, um diesen zur aktiven Führung der Bildaufnahmevorrichtung 13 in eine der Auslösestellungen aufzufordern. Hierzu weist die Bildaufnahmevorrichtung 13 ein nicht dargestelltes Display auf, das Anweisungen grafisch ausgeben kann. Beispielsweise kann der Benutzer durch Pfeile aufgefordert werden, die Bildaufnahmevorrichtung 13 weiter nach rechts oder links zu bewegen, weiter nach oben oder unten zu kippen, näher an das oder weiter weg vom Podest 1 zu führen oder die Bildaufnahmevorrichtung 13 höher oder tiefer zu halten. Zusätzlich oder alternativ kann die Bildaufnahmevorrichtung 13 einen Lautsprecher aufweisen, über den die Anweisungen akustisch wiedergegeben werden. Sobald die Bildaufnahmevorrichtung 13 erkennt, dass ihr relativer Standort und ihre Ausrichtung zum Podest 1 eine der Auslösestellungen eines ersten Bildes beziehungsweise eines der nächsten Bilder entspricht, nimmt diese automatisch den entsprechenden Fuß 2 mit der durch die vorgegebene Auslösestellung bestimmten Ansicht auf.

In bevorzugter Weise führt die Bildaufnahmevorrichtung 13 den Benutzer zu den einzelnen Auslösestellungen in der folgenden Reihenfolge: rechter Fuß von vorn, linker Fuß von vorn, linker Fuß von hinten, rechter Fuß von hinten.

Nachdem beide zu vermessenden Füße 2 aus allen für das dreidimensionale Modell erforderlichen Auslösestellungen fotografiert wurden, werden die digitalen Bilddateien von der Bildaufnahmevorrichtung 13 an eine Verarbeitungsvorrichtung 14 des Messsystems in verschlüsselter Form drahtlos übertragen. Die Bilddateien weisen in an sich bekannter Weise Metadaten auf, die hier auch Informationen über die jeweilige Auslösestellung umfassen. Die Bilddateien können zur Prozessoptimierung ebenso nach jeder Bildaufnahme einzeln übertragen werden. Die Bildaufnahmevorrichtung 13 und die Verarbeitungsvorrichtung 14 sind hier im selben lokalen Netzwerk eingebunden, wobei es sich bei der Verarbeitungsvorrichtung 14 um einen handelsüblichen PC handeln kann, der beispielsweise in einem Ladenlokal des Schuhfachgeschäfts aufgestellt ist, und mit einer Vielzahl von Bildaufnahmegeräten 13 kommuniziert. Die Verarbeitungsvorrichtung 14 kann aber auch ein zentraler Server sein, der mit mehreren Bildaufnahmevorrichtungen 13 an unterschiedlichen Orten über eine Internetverbindung verbunden sein kann. Alternativ kann die Verarbeitungsvorrichtung insbesondere mit der Bildaufnahmevorrichtung 13 in dem handgehaltenen Tablett-PC oder dem Smartphone integriert sein.

Die Verarbeitungsvorrichtung 14 berechnet die dreidimensionalen Modelle der beiden Füße 2 mittels der vorher aufgenommenen Fotos, auf denen die Konturen der Füße und die lediglich teilweise durch den jeweiligen Fuß 2 überdeckten Referenzmuster 10, 12 erkennbar sind. Aus den Bilddateien der verschiedenen Ansichten der Füße 2 im durch die Referenzmuster 10, 12 vorgegebenen Bildkoordinatensystem wird für jeden Fuß 2 mit Hilfe der vorgegebenen Auslösestellungen ein dreidimensionales Modell im Weltkoordinatensystem rückprojiziert. Aus den Bilddaten werden Punktwolken im Weltkoordinatensystem errechnet, woraus sich unter anderem die Maße der Füße 2 in Höhe, Breite und Tiefe ergeben.

Die biometrischen Daten der Füße 2 und die generierten dreidimensionalen Fußmodelle werden von der Verarbeitungsvorrichtung 14 an eine Auswertevorrichtung 15 des Messsystems übertragen. Die Auswertevorrichtung 15 ist hier ein über eine Internetleitung angeschlossener zentraler Server, auf welchem eine Vielzahl an Datensätzen von Schuhen hinterlegt ist, die beispielsweise Schuhgrößen und dreidimensionale Schuhmodelle umfassen. Die Auswertevorrichtung 15 gleicht die bestimmten biometrischen Daten der vermessenen Füße 2 mit den hinterlegten Datensätzen ab und übermittelt eine Trefferliste mit zu den biometrischen Daten passenden Schuhen über die Verarbeitungsvorrichtung 14 an eine Ausgabevorrichtung 16. Die Ausgabevorrichtung 16 ist mit der Bildaufnahmevorrichtung 13 in einer baulichen Einheit zusammengefasst, die als tragbares Handgerät, hier als Tablet-PC ausgebildet ist. Auf dem gemeinsam genutzten Display werden produktspezifische Informationen zu den in der Trefferliste aufgeführten Schuhen angezeigt, welche durch den Schuhverkäufer direkt dem Kunden präsentiert werden können.

### Bezugszeichenliste

- 1: Podest
- 2: Fuß
- 3: Bodenplatte
- 4: Wandelement
- 5: Längsenden
- 6: Spiegel
- 7: reflektierende Spiegelfläche
- 8: Oberfläche
- 9: Beschichtung
- 10: bodenseitiges Referenzmuster
- 11: Außenseite
- 12: wandseitiges Referenzmuster
- 13: Bildaufnahmevorrichtung
- 14: Verarbeitungsvorrichtung
- 15: Auswertevorrichtung
- 16: Ausgabevorrichtung

## Patentansprüche

1. Messverfahren zur Bestimmung biometrischer Daten menschlicher Füße, umfassend die folgenden Schritte:
- Bereitstellen eines Referenzobjektes (1), das wenigstens ein Referenzmuster (10, 12) aufweist,
- Bereitstellen einer frei beweglichen Bildaufnahmevorrichtung (13),
- Automatisches Erkennen des relativen Standortes und der Ausrichtung der Bildaufnahmevorrichtung (13) zum Referenzobjekt (1) anhand des wenigstens einen Referenzmusters (10, 12), **dadurch gekennzeichnet,**
**dass** das Messverfahren weiterhin folgende Schritte umfasst:
- Automatisches Aufnehmen mehrerer Bilder durch die Bildaufnahmevorrichtung (13), wobei jedes einzelne Bild nur dann aufgenommen wird, wenn der relative Standort und die Ausrichtung der Bildaufnahmevorrichtung (13) zum Referenzobjekt (1) einer für das jeweilige Bild vorgegebenen Auslösestellung entspricht,
- Bestimmung biometrischer Daten des wenigstens einen Fußes (2) mittels der aufgenommenen Bilder.

2. Messverfahren nach Anspruch 1,
**dadurch gekennzeichnet,**
**dass** der Schritt der Bestimmung der biometrischen Daten des wenigstens einen Fußes (2) mittels der aufgenommenen Bilder zumindest die folgenden Teilschritte umfasst:
- Generierung zumindest eines dreidimensionalen Modells wenigstens eines auf dem Referenzobjekt (1) platzierten Fußes auf Basis der aufgenommenen Bilder durch die Verarbeitungsvorrichtung (14), und
- Bestimmung der biometrischen Daten des wenigstens einen Fußes anhand des zumindest einen dreidimensionalen Modells durch die Verarbeitungsvorrichtung (14).

3. Messverfahren nach Anspruch 1 oder 2,
**dadurch gekennzeichnet,**
**dass** weiterhin folgender Schritt vorgesehen ist:
- Ausgabe von Anweisungen zur Führung der Bildaufnahmevorrichtung (13) zur jeweiligen Auslösestellung.

4. Messverfahren nach Anspruch 3,
**dadurch gekennzeichnet,**
**dass** vor der Ausgabe der jeweiligen Anweisung zur Führung der Bildaufnahmevorrichtung (13) zur jeweiligen Auslösestellung folgende Schritte vorgesehen sind:
- Automatisches Erkennen des relativen Standorts und der Ausrichtung der Bildaufnahmevorrichtung (13) zum Referenzobjekt (1) anhand des wenigstens einen Referenzmusters (10, 12),
- Berechnen eines Weges vom erkannten relativen Standort mit der erkannten Ausrichtung zur jeweiligen Auslösestellung.

5. Messverfahren nach Anspruch 3 oder 4,
**dadurch gekennzeichnet,**
**dass** die Anweisungen zur Führung des Benutzers über optische oder akustische Anzeigemittel erfolgen.

6. Messverfahren nach einem der vorherigen Ansprüche,
**dadurch gekennzeichnet,**
**dass** weiterhin folgende Schritte vorgesehen sind:
- Abgleichung der bestimmten biometrischen Daten des wenigstens einen Fußes (2) mit Datensätzen von Schuhen, die zumindest Angaben über Schuhgrößen oder Schuhformen diverser Schuhe umfassen, durch die Auswertevorrichtung (14), und
- Übermittlung einer Trefferliste mit zu den biometrischen Daten passenden Schuhen von der Auswertevorrichtung (14) an die Ausgabevorrichtung (16), und
- Wiedergabe von Informationen zu den in der Trefferliste aufgeführten Schuhen.

7. Messverfahren nach einem der vorherigen Ansprüche,
**dadurch gekennzeichnet,**
**dass** das Referenzobjekt ein Podest (1) umfasst, wobei das Podest (1) eine Bodenplatte (3), auf der wenigstens ein zu vermessender menschlicher Fuß (2) platzierbar ist, mit dem geometrischen bodenseitigen Referenzmuster (10) und ein quer zur Bodenplatte (3) angeordnetes Wandelement (4) mit dem geometrischen wandseitigen Referenzmuster (12) aufweist.

8. Messverfahren nach Anspruch 7,
**dadurch gekennzeichnet,**
**dass** das Wandelement (4) und die Bodenplatte (3) in einem Winkel zueinander angeordnet sind, der im Bereich zwischen 70 und 140 Grad liegt.

9. Messverfahren nach Anspruch 7 oder 8,
**dadurch gekennzeichnet,**
**dass** die Bodenplatte (3) und/oder das Wandelement (4) eine retroreflektierende Oberfläche (8) aufweist.

10. Messverfahren nach einem der Ansprüche 7 bis 9,
**dadurch gekennzeichnet,**
**dass** wenigstens ein quer zur Bodenplatte (3) angeordneter Spiegel (6) vorgesehen ist, wobei eine reflektierende Spiegelfläche (7) des Spiegels (6) dem wandseitigen Referenzmuster (12) gegenüberliegend angeordnet ist.

11. Messverfahren nach Anspruch 10,
**dadurch gekennzeichnet,**
**dass** der wenigstens eine Spiegel (6) und die Bodenplatte (3) in einem Winkel zueinander angeordnet sind, der im Bereich zwischen 70 und 160 Grad liegt.

12. Messverfahren nach einem der Ansprüche 7 bis 11,
**dadurch gekennzeichnet,**
**dass** das Podest (1) zu einer durch das Wandelement (4) aufgespannten Ebene (E) symmetrisch ausgebildet ist.

13. Messverfahren nach einem der vorherigen Ansprüche,
**dadurch gekennzeichnet,**
**dass** eine Verarbeitungsvorrichtung (14) bereitgestellt wird, die aus den Bildern ein dreidimensionales Modell des wenigstens einen Fußes (2) generiert und anhand des Modells biometrische Daten des wenigstens einen Fußes (2) bestimmt,
eine Auswertevorrichtung (15) bereitgestellt wird, die die ermittelten biometrischen Daten mit Datensätzen von Schuhen anhand hinterlegter Schuhgrößen und/oder Schuhformen abgleicht und die zu den ermittelten biometrischen Daten passenden Schuhe identifiziert, und
eine Ausgabevorrichtung (16) bereitgestellt wird, die Informationen zu den passenden Schuhen wiedergibt.

14. Messverfahren nach Anspruch 13,
**dadurch gekennzeichnet,**
**dass** die Bildaufnahmevorrichtung (13) und die Ausgabevorrichtung (16) eine tragbar ausgebildete bauliche Einheit bilden und die Verarbeitungsvorrichtung (14) sowie die Auswertevorrichtung (15) baulich von der Bildaufnahmevorrichtung (13) und der Ausgabevorrichtung (16) getrennt sind.

## Claims

1. Measuring method for determining biometric data of human feet, comprising the following steps:
- providing a reference object (1), which has at least one reference pattern (10, 12),
- providing a freely moveable image recording device (13),
- automatically detecting the relative position and the alignment of the image recording device (13) relative to the reference object (1) using the at least one reference pattern (10, 12),
**characterized in**
**that** the measuring method further comprising following steps:
- automatically recording several images by the image recording device (13), wherein each individual image is only recorded, when the relative position and the alignment of the image recording device (13) relative to the reference object (1) corresponds to a triggering position predetermined for the respective image,
- determining biometric data of the at least one foot (2) by means of the recorded images.

2. Measuring method according to claim 1,
**characterised in**
**that** the step of determining the biometric data of the at least one foot (2) by means of the recorded images comprises at least the following partial steps:
- generating at least one three-dimensional model of the at least one foot placed on the reference object (1) by a processing device (14) on the basis of the recorded images, and
- determining the biometric data of the at least one foot by the processing device (14) using the at least one three-dimensional model.

3. Measuring method according to claim 1 or 2,
**characterised in**
**that**, furthermore, the following step is provided:
- putting out instructions for transferring an image recording device (13) to the respective triggering position.

4. Measuring method according to claim 3,
**characterised in**
**that** before the putting out of the respective instruction for transferring the image recording device (13) to the respective triggering point, the following steps are provided:
- automatically detecting the relative position and of the alignment of the image recording device (13) relative to the reference object (1) by using the at least one reference pattern (10, 12),
- calculating a path from the detected relative position with the detected alignment to the respective triggering position.

5. Measuring method according to claim 3 or 4,
**characterised in**
**that** the instructions for guiding the user is carried out via optical or acoustic indication elements.

6. Measuring method according to one of the preceding claims,
**characterised in**
**that**, furthermore, the following steps are provided:
- comparing the determined biometric data of the at least one foot (2) with sets of data of shoes, which comprise at least information of the shoe sizes or of shoe shapes of various shoes, by an evaluation device (15), and
- transferring a hit list with shoes fitting the biometric data from the evaluation device (15) to an out-put device (16), and
- reproducing information relating to the shoes listed in the hit list.

7. Measuring method according to one of the preceding claims,
**characterised in**
**that** the reference object comprises a base plate (3), on which at least one to be measured human foot (2) is placeable, having a geometric base-sided reference pattern (10),
and a wall element (4), being arranged transversally to the base plate (3) and having a geometric wall-sided reference pattern (12).

8. Measuring method according to claim 7,
**characterised in**
**that** the wall element (4) and the base plate (3) are arranged at an angle to each other, which is in the range between 70 and 140 degrees.

9. Measuring method according to claim 7 or 8,
**characterised in**
**that** the base plate (3) and/or the wall element (4) has/have a retro-reflective surface (8).

10. Measuring method according to one of the claims 7 to 9,
**characterised in**
**that** at least one mirror (6) being arranged transversally to the base plate (3) is provided, wherein a reflective mirror face (7) of the mirror (6) is arranged opposite to the wall-sided reference pattern (12).

11. Measuring method according to claim 10,
**characterised in**
**that** the at least one mirror (6) arranged at an angel to the base plate (3), which is in the range between 70 und 160 degrees.

12. Measuring method according to one of the claims 7 to 11,
**characterised in**
**that** the pedestal (1) is formed symmetrically to a plane (E) formed by the wall element (4).

13. Measuring method according to one of the preceding claims,
**characterised in**
**that** a processing device (14) is provided, which generates a three-dimensional model of the at least one foot (2) from the images and determines biometric data of the at least one foot (2) by means of the model,
an evaluation device (15) is provided, which compares the determined biometric data with sets of data of shoes using stored shoe sizes and/or shoe shapes and identifies the shoes fitting to the determined biometric data, and an out-put device (16) is provided, which reproduces information with regard to the fitting shoes.

14. Measuring method according claim 13,
**characterised in**
**that** the image recording device (13) and the out-put device (16) form a portable structural unit and that the processing device (14) as well as the evaluation device (15) are structurally separately formed from the image recording device (13) and the out-put device (16).

## Revendications

1. Procédé de mesure pour déterminer des données biométriques de pieds humains, comprenant les étapes suivantes :
- préparation d'un objet de référence (1), qui comporte au moins un modèle de référence (10,11),
- préparation d'un dispositif de prise de vues (13) se déplaçant librement,
- identification automatique de la position relative et de l'orientation du dispositif de prise de vues (13) par rapport à l'objet de référence (1) à l'aide d'au moins un modèle de référence (10,12), **caractérisé en ce que**
le procédé de mesure comprend en plus les étapes suivantes :
- enregistrement automatique de plusieurs images par le dispositif de prise de vues (13), chaque image individuelle n'étant ensuite prise que si la position relative et l'orientation du dispositif de prise de vues (13) par rapport à l'objet de référence (1) correspond à une position de déclenchement prédéfinie pour l'image respective,
- détermination des données biométriques d'au moins un pied (2) au moyen des images enregistrées.

2. Procédé de mesure selon la revendication 1, **caractérisé en ce que**
- l'étape de détermination des données biométriques d'au moins un pied (2) au moyen des images enregistrées comprend au moins les parties d'étape suivantes :
- génération d'au moins un modèle tridimensionnel d'au moins un pied positionné sur l'objet de référence (1) sur la base des images enregistrées par le dispositif de prise de vues (14), et
- détermination des données biométriques d'au moins un pied à l'aide d'au moins un modèle tridimensionnel par le dispositif de traitement (14),

3. Procédé de mesure selon la revendication 1 ou 2, **caractérisé en ce que**
l'étape suivante est en plus prévue :
- édition d'instructions pour guider le dispositif de prise de vues (13) à la position respective de déclenchement.

4. Procédé de mesure selon la revendication 3, **caractérisé en ce qu'**
- avant d'éditer l'instruction respective pour guider le dispositif de prise de vues (13) à la position de déclenchement respective, les étapes suivantes sont prévue :
- identification automatique de la position relative et de l'orientation du dispositif de prise de vues (13) par rapport à l'objet de référence (1) à l'aide d'au moins un modèle de référence (10,12),
- calcul d'une trajectoire depuis la position relative identifiée avec l'orientation identifiée par rapport à la position de déclenchement respective.

5. Procédé de mesure selon la revendication 3 ou 4, **caractérisé en ce que**
les instructions pour guider l'utilisateur eu égard aux moyens d'affichage optiques ou acoustiques ont lieu.

6. Procédé de mesure selon l'une quelconque des revendications précédentes,
**caractérisé en ce que**
les étapes suivantes sont en plus prévue
- harmonisation des données biométriques définies d'au moins un pied (2) avec jeux de données des chaussures, qui comprennent au moins des indications sur les pointures ou les formes des chaussures de diverses chaussures par le dispositif d'exploitation (14), et
- transmission d'une liste de résultats avec les chaussures convenant aux données biométriques du dispositif d'exploitation (14) au dispositif d'édition (16), et
- restitution des informations aux chaussures mentionnées dans la liste des résultats.

7. Procédé de mesure selon l'une quelconque des revendications précédentes,
**caractérisé en ce que**
l'objet de référence comprend un socle (1), le socle (1) comprenant une plaque de sol (3) sur laquelle au moins un pied humain (2) à mesurer peut être placé, avec le modèle de référence (10) géométrique côté sol et un élément de cloison (4) disposé transversalement à la plaque de sol (3) avec le modèle de référence (12) géométrique côté cloison.

8. Procédé de mesure selon la revendication 7, **caractérisé en ce que**
1' élément de cloison (4) et la plaque de sol (3) sont disposés l'un par rapport à l'autre dans un angle qui se situe dans une plage variant entre 70 et 140 degrés.

9. Procédé de mesure selon la revendication 7 ou 8, **caractérisé en ce que**
La plaque de sol (3) et/ou l'élément de cloison (4) comporte une surface rétroréflectrice.

10. Procédé de mesure selon l'une quelconque des revendications 7 à 9,
**caractérisé en ce qu'**
au moins un miroir (6) disposé transversalement à la plaque de sol (3) est prévu, une surface de miroir réflectrice (7) du miroir (6) étant disposée opposée au modèle de référence (12) côté cloison.

11. Procédé de mesure selon la revendication 10, **caractérisé en ce qu'**
au moins un miroir (6) et la plaque de sol (3) sont disposés l'un par rapport à l'autre dans un angle se situant entre 70 et 160 degrés.

12. Procédé de mesure selon l'une quelconque des revendications 7 à 11,
**caractérisé en ce que**
le socle (1) est constitué symétriquement par rapport à un plan (E) s'étendant à travers l'élément de cloison (4).

13. Procédé de mesure selon l'une quelconque des revendications précédentes,
**caractérisé en ce qu'**
un dispositif de traitement (14) est fourni, qui génère un modèle tridimensionnel d'au moins un pied à partir des images et détermine à l'aide du modèle des données biométriques d'au moins un pied (2),
un dispositif d'exploitation (15) est fourni, qui harmonise les données biométriques déterminées avec des jeux de sonnées des chaussures à l'aide des pointures et/ou formes de chaussures mémorisées et qui identifie les chaussures convenant aux données biométriques déterminées, et
un dispositif d'édition (16) est fourni, qui restitue les informations aux chaussures appropriées.

14. Procédé de mesure selon la revendication 13, **caractérisé en ce que**
Le dispositif de prise de vues (13) et le dispositif d'édition (16) forment une unité structurale constituée de façon portative et le dispositif de traitement (14) ainsi que le dispositif d'exploitation (15) sont structuralement séparés du dispositif de prise de vues (13) et du dispositif d'édition (16).
